(19) Europäisches Patentamt
European Patent Office
Office européen des brevets

(11) Veröffentlichungsnummer: 0 507 140 A2

(12) **EUROPÄISCHE PATENTANMELDUNG**

(21) Anmeldenummer: **92104534.0**

(22) Anmeldetag: **17.03.92**

(51) Int. Cl.5: **C07F 9/40**, A01N 57/04, C07F 9/177, C07F 9/24, C07F 9/42, C07F 9/14, C07F 9/20, C07C 35/06, C07C 35/48

(30) Priorität: **30.03.91 DE 4110486**

(43) Veröffentlichungstag der Anmeldung: **07.10.92 Patentblatt 92/41**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Krüger, Bernd-Wieland, Dr.**
**Unterboschbach 19**
**W-5060 Bergisch Gladbach 2(DE)**
Erfinder: **Negele, Michael, Dr.**
**Wolfskaul 6**
**W-5000 Köln 80(DE)**
Erfinder: **Hartwig, Jürgen, Dr.**
**Franz-Esser-Strasse 44**
**W-5090 Leverkusen 3(DE)**
Erfinder: **Erdelen, Christoph, Dr.**
**Unterbüscherhof 22**
**W-5653 Leichlingen 1(DE)**
Erfinder: **Stendel, Wilhelm, Dr.**
**In den Birken 55**
**W-5600 Wuppertal 1(DE)**

(54) **O-Halogencyclopentyl-S-alkyl-(di)thiophosphor(phosphon)-säureester-Derivate, Verfahren zu ihrer Herstellung und ihre Verwendung als Schädlingsbekämpfungsmittel.**

(57) O-Halogencyclopentyl-S-alkyl-(di)thiophosphor(phosphon)säureester-Derivate der allgemeinen Formel (I)

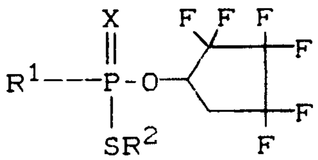

(I)

in welcher

$R^1$ für Alkyl, Alkoxy oder den Rest

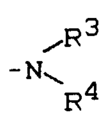

steht, wobei

$R^3$ für Wasserstoff oder Alkyl steht und

$R^4$ für Wasserstoff, Alkyl, -COH (Formyl) oder gegebenenfalls durch Halogen substituiertes -CO-Alkyl (Acyl) steht,

EP 0 507 140 A2

$R^2$ für Alkyl oder Alkoxyalkyl steht und

X für Sauerstoff oder Schwefel steht,

welche als Schädlingsbekämpfungsmittel verwendet werden können.

Die Erfindung betrifft neue O-Halogencyclopentyl-S-alkyl-(di)thiophosphor(phosphon)säureester-Derivate, mehrere Verfahren zu ihrer Herstellung und ihre Verwendung als Schädlingsbekämpfungsmittel, insbesondere als Insektizide, Akarizide und Nematizide.

Es ist bereits bekannt, daß bestimmte O-Halogencyclobutyl-S-(alkyl)-(di)thiophosphorsäureester, wie z.B. Thiophosphorsäure-S-propyl-O-ethyl-O-(3,3,2-trifluor-2-chlor-cyclobutyl)-triester zur Schädlingsbekämpfung verwendet werden können (vgl. US-Patentschrift 4 973 583).

Die insektizide, akarizide und nematizide Wirkung der bekannten Verbindungen ist jedoch, insbesondere bei niedrigen Wirkstoffkonzentrationen und Aufwandmengen, nicht immer bei allen Problemstellungen voll zufriedenstellend.

Es wurden nun neue O-Halogencyclopentyl-S-alkyl-(di) thiophosphor(phosphon)-säureester-Derivate der allgemeinen Formel (I)

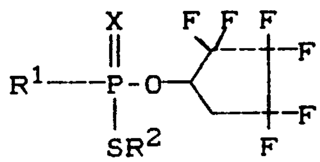

(I)

gefunden, in welcher

$R^1$ für Alkyl, Alkoxy oder den Rest

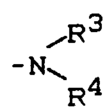

steht, wobei

$R^3$ für Wasserstoff oder Alkyl steht und

$R^4$ für Wasserstoff, Alkyl, -COH (Formyl) oder gegebenenfalls durch Halogen substituierter -CO-Alkyl (Acyl) steht,

$R^2$ für Alkyl oder Alkoxyalkyl steht und

X für Sauerstoff oder Schwefel steht.

Die Verbindungen der Formel (I) besitzen gegebenenfalls ein oder mehrere Asymmetrie-Zentren. Sie können daher in verschiedenen Isomerenformen vorliegen, die in unterschiedlichen Mengenverhältnissen anfallen können. Die Erfindung betrifft sowohl die Isomerengemische als auch die einzelnen Isomeren.

Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß man die neuen O-Halogencyclopentyl-S-alkyl-(di)thiophosphor-(phosphon)säureester-Derivate der Formel (I) erhält,

wenn man

a) Thio- oder Dithiophosphorsäureesterhalogenide der Formel (II)

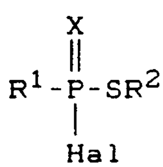

(II)

in welcher

$R^1$, $R^2$ und X die oben angegebene Bedeutung haben und

Hal für Halogen steht,

mit Cyclopentanol-Derivaten der Formel (III)

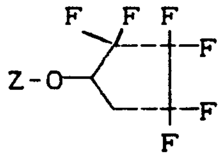

(III)

in welcher

Z für Wasserstoff oder ein Äquivalent eines Alkalimetallions steht, gegebenenfalls in Gegenwart von Verdünnungsmitteln und gegebenenfalls in Gegenwart von Basen umsetzt,

oder wenn man

b) zur Herstellung von Verbindungen der Formel (I), in welchen $R^1$ für Alkoxy oder $-NR^3R^4$ steht, in einem ersten Reaktionsschritt Säurehalogenide der Formel (IV)

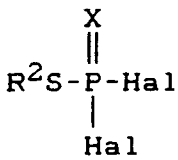

(IV)

in welcher

X, $R^2$ und Hal die oben angegebene Bedeutung besitzen,

mit Verbindungen der Formel

$R^5$-H (V)

in welcher

$R^5$ für Alkoxy oder den Rest $-NR^3R^4$ steht, wobei $R^3$ und $R^4$ die oben angegebene Bedeutung haben,

gegebenenfalls in Gegenwart von Lösungsmitteln und gegebenenfalls in Gegenwart von Basen umsetzt und das erhaltene Produkt der Formel (VI)

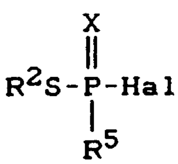

(VI)

in welcher

X, $R^2$, $R^5$ und Hal die oben angegebene Bedeutung haben,

anschließend in einem zweiten Reaktionsschritt mit Verbindungen der Formel (III) gegebenenfalls in Gegenwart von Verdünnungsmitteln und gegebenenfalls in Gegenwart von Basen, umsetzt,

oder wenn man

c) in einem ersten Reaktionsschritt Verbindungen der Formel (VII)

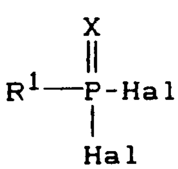

(VII)

in welcher

$R^1$, X und Hal die oben angegebene Bedeutung haben,
mit Verbindungen der Formel (III), gegebenenfalls in Gegenwart von Verdünnungsmitteln und gegebenenfalls in Gegenwart von Basen, umsetzt und die erhaltenen Verbindungen der Formel (VIII)

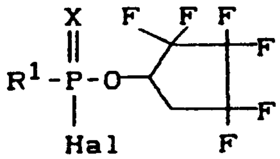

(VIII)

in welcher
$R^1$, X und Hal die oben angegebene Bedeutung haben,
in einem zweiten Reaktionsschritt mit Verbindungen der Formeln (IXa) oder (IXb)

$Z^1$-SH (IXa)

oder

$(Z^1)_2S$ (IXb)

in welcher
$Z^1$ für ein Äquivalent eines Alkalimetallions steht,
gegebenenfalls in Gegenwart eines Verdünnungsmittels, umsetzt und die erhaltenen Verbindungen der Formel (X)

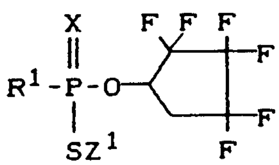

(X)

in welcher
$R^1$, X und $Z^1$ die oben angegebene Bedeutung haben,
mit Verbindungen der Formel (XI)

$R^2$-Hal (XI)

in welcher
$R^2$ und Hal die oben angegebene Bedeutung haben,
gegebenenfalls in Gegenwart eines Verdünnungsmittels und gegebenenfalls in Gegenwart einer Base, umsetzt,
oder wenn man
d) zur Herstellung von Verbindungen der Formel (I), in welchen $R^1$ für Alkyl und X für Schwefel stehen, in einem ersten Reaktionsschritt, Verbindungen der Formel (XII)

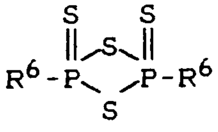

(XII)

in welcher

$R^6$ für Alkyl steht,

mit Cyclopentanol-Derivaten der Formel (III), gegebenenfalls in Gegenwart von Verdünnungsmitteln und gegebenenfalls in Gegenwart von Basen, umsetzt, und die erhaltenen Verbindungen der Formel (XIII)

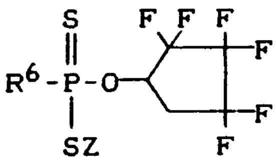

(XIII)

in welcher

$R^6$ und Z die oben angegebene Bedeutung haben,

in einem zweiten Schritt mit Verbindungen der Formel (XI), gegebenenfalls in Gegenwart von Verdünnungsmitteln und gegebenenfalls in Gegenwart von Basen, umsetzt,

oder wenn man

e) zur Herstellung von Verbindungen der Formel (I), in welchen $R^1$ für $-NR^3R^4$ steht, in einem ersten Schritt Verbindungen der Formel (IV) mit Verbindungen der Formel (III), gegebenenfalls in Gegenwart von Verdünnungsmitteln und gegebenenfalls in Gegenwart von Basen, umsetzt und in einem zweiten Schritt die erhaltenen Verbindungen der Formel (XIV)

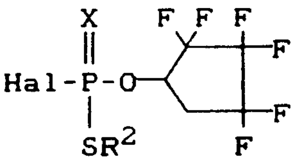

(XIV)

in welcher

X, $R^2$ und Hal die oben angegebene Bedeutung haben,

mit Verbindungen der Formel (XV)

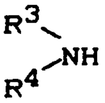

(XV)

in welcher

$R^3$ und $R^4$ die oben angegebene Bedeutung haben,

gegebenenfalls in Gegenwart eines Verdünnungsmittels und gegebenenfalls in Gegenwart einer Base, umsetzt,

oder wenn man

f) zur Herstellung von Verbindungen der Formel (I), in welcher $R^4$ für Formyl oder Acyl steht, Verbindungen der Formel (XVI)

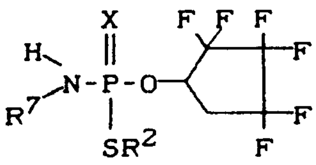

(XVI)

in welcher

X und $R^2$ die oben angegebene Bedeutung haben und

$R^7$ für Wasserstoff oder Alkyl steht,

mit Verbindungen der Formel (XVII)

$R^8$-CO-Y (XVII)

in welcher

$R^8$ für Wasserstoff oder gegebenenfalls durch Halogen substituiertes Alkyl steht und

Y für Halogen oder eine andere bei Acylierungsreaktionen abgehende Gruppe steht,

gegebenenfalls in Gegenwart eines Verdünnungsmittels oder gegebenenfalls in Gegenwart einer Base, umsetzt.

Weiterhin wurde gefunden, daß die neuen Verbindungen der allgemeinen Formel (I) eine sehr gute Wirksamkeit als Schädlingsbekämpfungsmittel, insbesondere gegen unerwünschte Arthropoden, wie Insekten und Spinnmilben sowie gegen Nematoden aufweisen. Die neuen Verbindungen stellen somit eine wertvolle Bereicherung der Technik dar.

In den allgemeinen Formeln bedeutet der Rest

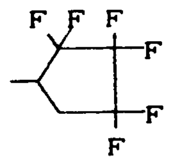

den 2,2,3,3,4,4-Hexafluorcyclopentylrest der Formel

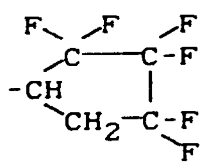

Die in den allgemeinen Formeln aufgeführten Reste werden, wie folgt, erläutert:

Alkyl bedeutet geradkettiges oder verzweigtes Alkyl mit vorzugsweise 1 bis 6, besonders bevorzugt 1 bis 4 und ganz besonders bevorzugt 1 bis 3 Kohlenstoffatomen, wobei Methyl, Ethyl, n- und i-Propyl sowie n-, i-, sec- und t-Butyl speziell genannt seien.

Für die Alkylteile von -CO-Alkyl, Alkoxy und Alkoxyalkyl gelten die für Alkyl angegebenen Erläuterungen. Als CO-Alkyl sollen speziell -CO-$C_1$-$C_4$-Alkyl und insbesondere -$COCH_3$ sowie -$COC_2H_5$ hervorgehoben werden. Als Alkoxy sollen speziell $C_1$-$C_3$-Alkoxy, insbesondere Methoxy und Ethoxy hervorgehoben werden. Als Alkoxyalkyl sollten speziell $C_1$-$C_4$-Alkoxy-$C_1$-$C_2$-alkyl, insbesondere Methoxymethyl, Ethoxymethyl, Methoxyethyl und Ethoxyethyl hervorgehoben werden.

Das durch Halogen substituierte CO-Alkyl enthält ein oder mehrere, vorzugsweise 1 bis 5, besonders bevorzugt 1 bis 3 gleiche oder verschiedene Halogenatome, wobei speziell $COCF_3$ besonders hervorgehoben sei. Entsprechendes gilt auch für den Alkylrest $R^8$.

Halogen bedeutet Fluor, Chlor, Brom und Iod, vorzugsweise Chlor, Brom und Iod, besonders bevorzugt Chlor und Brom und ganz besonders bevorzugt Chlor. Im durch Halogen substituierten -CO-Alkyl bedeutet Halogen vorzugsweise Fluor, Chlor und/oder Brom, besonders bevorzugt Fluor und/oder Chlor und ganz besonders bevorzugt Fluor.

Als Alkalimetallionen werden Lithium-, Natrium- und Kaliumionen bevorzugt.

$R^1$ steht vorzugsweise für $C_1$-$C_6$-Alkyl, $C_1$-$C_6$-Alkoxy oder -$NR^3R^4$, worin

$R^3$ für Wasserstoff oder $C_1$-$C_6$-Alkyl steht und

$R^4$ für Wasserstoff, $C_1$-$C_6$-Alkyl, HCO- (Formyl) oder gegebenenfalls durch Halogen substituiertes -CO-$C_1$-$C_4$-Alkyl (Acyl) steht.

$R^2$ steht vorzugsweise für $C_1$-$C_6$-Alkyl oder $C_1$-$C_6$-Alkoxy-$C_1$-$C_6$-alkyl.

Erfindungsgemäß bevorzugt sind die Verbindungen der Formel (I), in welchen eine Kombination dieser vorstehend als vorzugsweise aufgeführten Bedeutungen vorliegt.

$R^1$ steht besonders bevorzugt für $C_1$-$C_4$-Alkyl, $C_1$-$C_4$-Alkoxy oder -$NR^3R^4$, worin

$R^3$ für Wasserstoff oder $C_1$-$C_4$-Alkyl steht und

$R^4$ für Wasserstoff, $C_1$-$C_4$-Alkyl, HCO- oder gegebenenfalls durch Halogen substituiertes -CO-$C_1$-$C_4$-Alkyl steht.

$R^2$ steht besonders bevorzugt für $C_1$-$C_4$-Alkyl oder $C_1$-$C_4$-Alkoxy-$C_1$-$C_4$-alkyl.

Erfindungsgemäß besonders bevorzugt sind die Verbindungen der Formel (I), in welchen eine Kombination dieser vorstehend als besonders bevorzugt aufgeführten Bedeutungen vorliegt.

$R^1$ steht ganz besonders bevorzugt für $C_1$-$C_3$-Alkyl, Methoxy, Ethoxy, $-NHCH_3$ oder $-NHC_2H_5$.

$R^2$ steht ganz besonders bevorzugt für $C_3$-$C_4$-Alkyl (insbesondere für n- und i-Propyl, n-Butyl, i-Butyl oder sec.-Butyl).

Erfindungsgemäß ganz besonders bevorzugt sind die Verbindungen der Formel (I), in welchen eine Kombination dieser vorstehend als ganz besonders bevorzugt aufgeführten Bedeutungen vorliegt.

Erfindungsgemäß besonders hervorgehoben werden die Verbindungen der Formel (I), in welchen X für Sauerstoff steht.

Die oben aufgeführten allgemeinen oder in Vorzugsbereichen aufgeführten Restedefinitionen bzw. Erläuterungen können untereinander, also auch zwischen den jeweiligen Vorzugsbereichen beliebig kombiniert werden. Sie gelten für die Vor- und Zwischenprodukte entsprechend.

Im einzelnen seien außer den bei den Herstellungsbeispielen genannten Verbindungen die folgenden O-Halogencyclopentyl-S-alkyl-(di)thiophosphor(phosphon)säureester-Derivate der allgemeinen Formel (I) beispielhaft genannt:

| $R^1$ | $R^2$ | X |
|---|---|---|
| $(C_2H_5)_2N$ | $-C_3H_7$-n | O |
| $(CH_3)_2N$ | $-C_3H_7$-n | O |
| $CH_3O$ | $-C_3H_7$-n | O |
| $CH_3O$ | $-\underset{CH_3}{\underset{\vert}{CH}}-C_2H_5$ | O |
| $CH_3O$ | $-C_3H_7$-n | S |
| $CH_3O$ | $-\underset{CH_3}{\underset{\vert}{CH}}-C_2H_5$ | S |
| $CH_3NH$ | $-C_3H_7$-n | O |
| $CH_3NH$ | $-\underset{CH_3}{\underset{\vert}{CH}}-C_2H_5$ | O |
| $C_2H_5NH$ | $-\underset{CH_3}{\underset{\vert}{CH}}-C_2H_5$ | O |
| $C_2H_5NH$ | $-C_3H_7$-n | S |
| $C_2H_5NH$ | $-\underset{CH_3}{\underset{\vert}{CH}}-C_2H_5$ | S |

Die als Ausgangsstoffe verwendeten Phosphor(n)säure-Derivate der allgemeinen Formel (II), (IV), (VII) und (XII) sind bekannt oder können nach bekannten Verfahren hergestellt werden (vgl. "Methoden der organischen Chemie" (Houben-Weyl) Bd. E2, 1982, Georg Thieme Verlag Stuttgart, New York, S. 300 ff. und S. 487 ff.).

Die Ausgangsstoffe der Formeln (VIII), (X), (XIII) und (XIV) sind neu und Teil der vorliegenden Erfindung. Sie werden im Verlaufe der Verfahrensvarianten c), d) bzw. e) erhalten. Ihre Isolierung und Reinigung erfolgt nach den üblichen Methoden, z.B. durch Destillation oder Chromatographie.

Die neuen Verbindungen der Formeln (VIII), (X) und (XIV) können unter der folgenden Formel (XVIII) zusammengefaßt werden

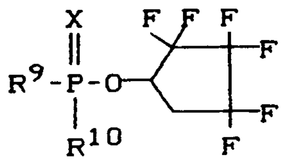

(XVIII)

in welcher

(a)

$R^9$ die Bedeutung von $R^1$ hat und

$R^{10}$ für Halogen oder $SZ^1$ steht oder

(b)

$R^9$ für Halogen steht und $R^{10}$ für $SR^2$ steht,

wobei $R^1$, $Z^1$ und $R^2$ die oben angegebene Bedeutung haben.

Die bei den erfindungsgemäßen Verfahren als Ausgangsstoffe verwendeten Cyclopentyl-Derivate der Formel (III) sind neu Sie können nach bekannten Methoden erhalten werden. Die Herstellung der Verbindung der Formel (III) in welcher Z für Wasserstoff steht kann beispielsweise durch Addition von Phenol oder 1-Propanol an Hexafluordichlorcyclopenten, Hydrierung und anschließende Etherspaltung gemäß folgenden Formelschema erfolgen:

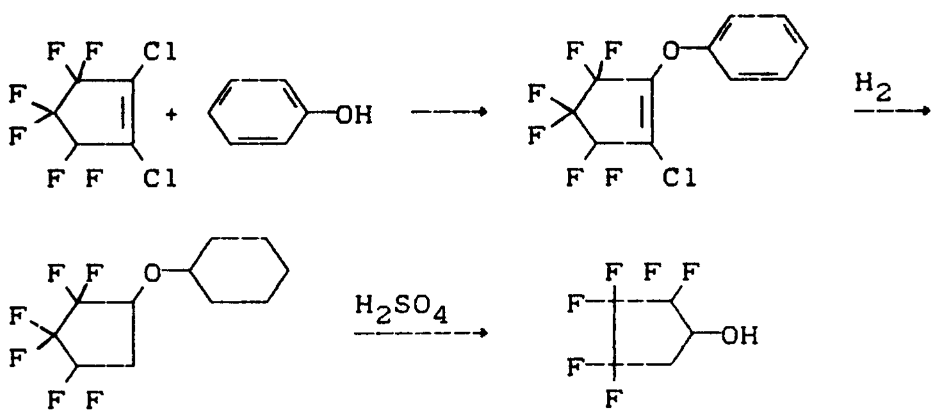


Die Alkali-Salze können nach den üblichen Methoden aus dem Alkohol erhalten werden.

Die übrigen als Ausgangsstoffe zu verwendenden Verbindungen sind bekannt oder können nach allgemein bekannten Methoden hergestellt werden.

Als Verdünnungsmittel für die erfindungsgemäße Verfahrensvariante a) kommen praktisch alle inerten organischen Verdünnungsmittel und ihre Mischungen in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethyl ether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon sowie Dimethylsulfoxid, Tetramethylensulfon und Hexamethylphosphorsäuretriamid.

Zur Durchführung der erfindungsgemäßen Verfahrensvariante a) setzt man auf 1 Mol Phosphor-Derivat der Formel (II) 1 bis 2 Mol, vorzugsweise 1,0 bis 1,8 Mol Cyclopentyl-Verbindung der Formel (III) ein.

Als Verdünnungsmittel für die erfindungsgemäßen Verfahrensvarianten b), c), d), e) und f) können praktisch alle inerten organischen Verdünnungsmittel verwendet werden. Vorzugsweise werden die Verdünnungsmittel verwendet, die im Zusammenhang mit der Beschreibung der erfindungsgemäßen Verfahrensvariante a) genannt wurden.

Die Verfahrensvarianten a), b), c), d), e) und f) können gegebenenfalls in Gegenwart von Basen durchgeführt werden, Als Basen können alle üblichen Basen Verwendung finden, Besonders bevorzugt werden Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Collidin, Triethylamin, Trimethylamin, Dimethylanilin, Dimethylbenzylamin, Tetraethylendiamin (DABCO) und Pyridin. Vorzugsweise werden die Basen in einer Menge hinzugefügt, die zum Abfangen des gebildeten Halogenwasserstoffes erforderlich ist.

Die erfindungsgemäßen Verfahrensvarianten a), b), c), d), e) und f) werden im allgemeinen bei Temperaturen zwischen -70 ° C und +150 ° C durchgeführt. Bevorzugt wird der Bereich zwischen -40 ° C und 110 ° C.

Die Umsetzungen werden im allgemeinen bei Normaldruck durchgeführt.

Zur Durchführung der erfindungsgemäßen Verfahrensvariante b) setzt man auf 1 Mol der Verbindung der Formel (IV) 1 bis 1,6 Mol, vorzugsweise 1 bis 1,4 Mol der Verbindung der Formel (V) und 1 bis 2, vorzugsweise 1 bis 1,8 Mol der Cyclopentyl-Verbindung der Formel (III) ein.

Zur Durchführung der erfindungsgemäßen Verfahrensvariante c) setzt man auf 1 Mol der Verbindung der Formel (VII) 1 bis 1,6 Mol, vorzugsweise 1 bis 1,4 Mol, der Cyclopentyl-Verbindung der Formel (III), 1 bis 2, vorzugsweise 1 bis 1,8 Mol, der Verbindungen der Formeln (IXa) bzw. (IXb) und 1 bis 2, vorzugsweise 1 bis 1,8 Mol, der Alkylhalogenide der Formel (XI) ein.

Zur Durchführung der Verfahrensvariante d) setzt man auf 1 Mol der Verbindung der Formel (XII) 2 bis 3 Mol, vorzugsweise 2 bis 2,5 Mol der Cyclopentyl-Verbindung der Formel (III) und 2 bis 3, vorzugsweise 2 bis 2,5 Mol der Alkylhalogenide der Formel (XI) ein.

Zur Durchführung der erfindungsgemäßen Verfahrensvariante e) setzt man auf 1 Mol der Verbindung der Formel (IV) 1 bis 2 Mol, vorzugsweise 1 bis 1,3 Mol der Cyclopentyl-Verbindung der Formel (III) und für den zweiten Reaktionsschritt auf 1 Mol des Monohalogenides der Formel (XIV) 1 bis 4 Mol, vorzugsweise 1 bis 1,5 Mol des Amins der Formel (XV) ein.

Zum Erhalt der Verbindungen der Formel (I), in welcher $R^1$ für Acyl- oder Formylamino steht, werden in Verfahrensvariante f) auf 1 Mol der Phosphorverbindung der Formel (XVI) 1 bis 2, vorzugsweise 1 bis 1,3 Mol der Verbindung der Formel (XVII) eingesetzt. Außer Carbonsäurehalogeniden, vorzugsweise den Chloriden, werden bei der Verfahrensvariante f) auch andere Carbonsäurederivate, wie Carbonsäureanhydride, z.B. Acetanhydrid vorteilhaft eingesetzt.

Die Aufarbeitung der erfindungsgemäßen Verbindungen geschieht nach üblichen Methoden. Die neuen Verbindungen fallen zum Teil in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung kann der Brechungsindex dienen.

Die erfindungsgemäßen Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich durch eine hohe insektizide, akarizide und vor allem nematizide Wirksamkeit aus. Sie lassen sich insbesondere gegen pflanzenschädigende Insekten, wie beispielsweise gegen die Raupen der Kohlschabe (Plutella maculipennis) oder gegen die Larven der Meerrettichblattkäfer (Phaedon cochleariae), sowie gegen pflanzenschädigende Milben, wie beispielsweise gegen die gemeine Spinnmilbe (Tetranychus urticae) einsetzen. Sie eignen sich daneben hervorragend zur Bekämpfung von Bodeninsekten und Nematoden und lassen sich beispielsweise zur Bekämpfung von Phorbia antiqua-Maden oder von Nematoden der Gattung Meloidogyne incognita einsetzen. Auch eine gute wurzelsystemische Wirkung, beispielsweise gegen Phaedon cochleariae-Larven ist hervorzuheben. Die nematizide Wirkung der erfindungsgemäßen Wirkstoffe läßt sich auch im in vitro-Test beispielsweise gegen endoparasitisch lebende Nematoden der Gattung Caenorhabditis elegans bestätigen.

Außerdem besitzen die erfindungsgemäßen Wirkstoffe der Formel (I) eine hohe Wirkung gegen Hygiene- und Vorratsschädlinge und lassen sich beispielsweise zur Bekämpfung der orientalischen Schabe (Blatta orientalis) oder zur Bekämpfung des gemeinen Kornkäfers (Sitophilus granarius) einsetzen. Darüber hinaus lassen sich die erfindungsgemäßen Wirkstoffe mit besonders gutem Erfolg zur Bekämpfung von parasitisch lebenden Warmblüterschädlingen, (sowie Ekto- als auch Endoparasiten), wie beispielsweise gegen die Larven der Goldfliege (Lucilia cuprina), gegen Rinderzecken (Boophilus microplus), gegen

Räudemilben (Psoroptes ovis), gegen Stechfliegen (Stomoxys calcitrans) oder gegen die Weideviehfliege (Musca autumnalis) einsetzen.

Daneben besitzen die erfindungsgemäßen Wirkstoffe der Formel (I) auch eine gute fungizide Wirksamkeit und lassen sich zur Bekämpfung von Pflanzenkrankheiten wie beispielsweise gegen den Erreger der Reisfleckenkrankheit (Pyricularia oryzae) oder gegen Schorf- und Botrytis-Erreger einsetzen.

In entsprechenden Aufwandmengen zeigen die erfindungsgemäßen Wirkstoffe der Formel (I) darüber hinaus eine herbizide Wirksamkeit.

Die erfindungsgemäßen Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmneben-Formulierungen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Verbindungen eignen sich in besonderer Weise auch zur Behandlung von generativem oder vegetativem Vermehrungsmaterial (Saatgut), wie Getreide, Mais, Kartoffel, Zwiebeln usw. Die Aufwandmengen liegen vorzugsweise bei 0,5 bis 20, insbesondere bei 1 bis 5 g je kg Vermehrungsmaterial.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken u.s.w. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer u.s.w. erreicht werden können.

Die Anwendung der erfindungsgemäßen Wirktoffe geschieht auf diesen Gebiet in bekannter Weise, wie durch äußerliche Anwendung in Form beispielsweise des Tauchens (Dippen), Sprühens (Sprayen), Aufgießens (pour-on and spot-on) und des Einpuderns.

Die Herstellung der erfindungsgemäßen Verbindungen soll anhand der folgenden Beispiele erläutert werden.

Beispiel 1

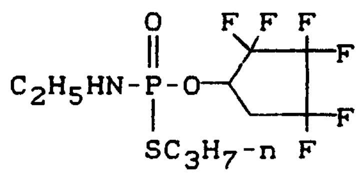

10 g (0,05 Mol) Thiophosphorsäure-dichlorid-S-propylester werden in 500 ml Hexan gelöst und zunächst zwischen 0 und -5 °C mit 10 ml (0,07 Mol) Triethylamin, dann mit 10 g (0,052 Mol) 2,2,3,3,4,4-Hexafluorcyclopentanol-1, gelöst in 25 ml Methylenchlorid, versetzt. Man rührt 2 Stunden bei 20 °C und tropft dann bei 20 °C 5 g (0,11 Mol) Ethylamin zur Reaktionsmischung. Zur Vervollständigung der Reaktion rührt man 24 h bei 20 °C, filtriert den Feststoff ab und destilliert das Lösungsmittel bei vermindertem Druck ab. Der Rückstand wird dann durch Filtration über Kieselgel (Cyclohexan:Essigsäureethylester = 7:3 Volumenteile) weiter gereinigt. Man erhält nach Abdestillieren des Lösungsmittels 8,5 g (47 % d. Th.) Thiophosphorsäure-O-(2,2,3,3,4,4-hexafluorcyclopentyl)-S-propyl-diester-N-ethylamid mit einem Brechungsindex $n_D^{20}$ = 1,4292.

Beispiel 2

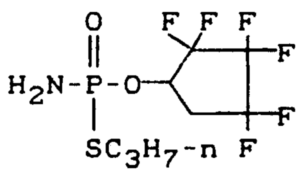

20 g (0,1 Mol) Thiophosphorsäure-dichlorid-S-propylester werden in 500 ml Hexan gelöst und zunächst zwischen 0 und -5 °C mit 20 ml (0,14 Mol) Triethylamin, dann mit 20 g (0,1 Mol) 2,2,3,3,4,4-Hexafluorcyclopentanol-1, gelöst in 50 ml Methylenchlorid, versetzt. Man rührt 2 Stunden bei 20 °C und

leitet dann bei 20 ° C Ammoniak ein (DC-Kontrolle). Zur Vervollständigung der Reaktion rührt man 24 h bei 20 ° C, filtriert den Feststoff ab und destilliert das Lösungsmittel bei vermindertem Druck ab. Der Rückstand wird dann durch Filtration über Kieselgel (Cyclohexan:Essigsäureethylester = 7:3 Volumenteile) weiter gereinigt. Man erhält nach Abdestillieren des Lösungsmittels 8,6 g (27 % d.Th.) Thiophosphorsäure-O-(2,2,3,3,4,4-hexafluorcyclopentyl)-S-propyldiester-amid mit einem Brechungsindex $n_D^{20}$ = 1,4390.

Beispiel 3

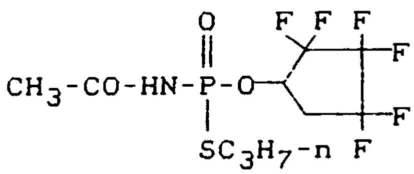

3,2 g (0,01 Mol) Thiophosphorsäure-O-(2,2,3,3,4,4-hexafluorcyclopentyl)-S-propyl-diester-amid werden in 50 ml Pyridin gelöst und bei 20 ° C mit 0,5 g 4-Pyrrolidinopyridin und 1 g Acetanhydrid (0,011 Mol) versetzt. Man rührt 24 h bei 40 ° C, destilliert dann das Pyridin bei vermindertem Druck ab und filtriert den Rückstand über Kieselgel (Cyclohexan:Essigsäureethylester = 7:3 Volumenteile). Man erhält nach Abdestillieren des Lösungsmittels 1,1 g (29 % d.Th.) Thiophosphorsäure-O-(2,2,3,3,4,4-hexafluorcyclopentyl)-S-propyldiesteracetamid mit einem Brechungsindex $n_D^{20}$ = 1,4751.

Beispiel 4

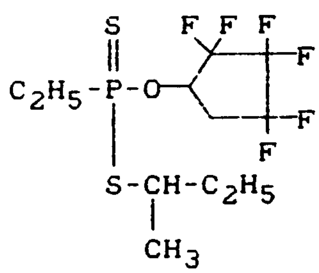

6,3 g (0,02 Mol) Ethanthiophosphonsäuredithioanhydrid werden in Toluol suspendiert und bei 20 ° C mit 7,8 g (0,04 Mol) 2,2,3,3,4,4-Hexafluorcyclopentanol-1, gelöst in 10 ml Toluol, versetzt. Man rührt 0,5 h bei 20 ° C, tropft dann bei 20 ° C 8 ml (0,058 Mol) Triethylamin zur Reaktionsmischung (exotherm bis ca. 40 ° C). Man rührt noch 2 h bei 50 ° C und fügt dann bei 20 ° C 9,1 g (0,066 Mol) sek.-Butylbromid hinzu. Zur Vervollständigung der Reaktion rührt man 24 h bei 80 ° C, gießt dann die Reaktionsmischung auf Wasser, extrahiert mit Methylenchlorid, trocknet mit Magnesiumsulfat, filtriert und destilliert das Lösungsmittel bei vermindertem Druck ab. Der Rückstand wird dann durch Filtration über Kieselgel (Cyclohexan:Essigsäureethylester = 7:3 Volumenteile) weiter gereinigt. Man erhält nach Abdestillieren des Lösungsmittels 9,4 g (63 % d.Th.) Ethanthiophosphonsäure-O-(2,2,3,3,4,4-hexafluorcyclopentyl)-S-(1-methylpropyl)-diester mit einem Brechungsindex $n_D^{20}$ = 1,4575.

Beispiel 5

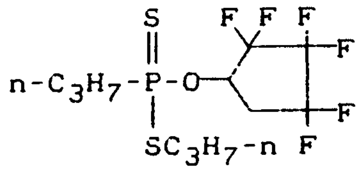

Analog Beispiel 4 erhält man den Propanthiophosphonsäure-O-(2,2,3,3,4,4-hexafluorcyclopentyl)-S-propyl-diester mit einem Brechungsindex $n_D^{20}$ = 1,4595.

Beispiel 6

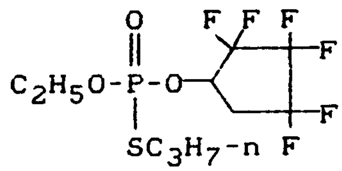

4 g (0,02 Mol) 2,2,3,3,4,4-Hexafluorcyclopentanol-1, gelöst in 50 ml Tetrahydrofuran, werden bei -70 °C mit 8 ml einer 2,5-molaren Butyllithium-Lösung in Hexan versetzt. Man erwärmt auf -20 °C und tropft dann 5 g (0,02 Mol) Thiophosphorsäure-chlorid-O-ethyl-S-propyl-diester hinzu. Zur Vervollständigung der Reaktion rührt man 24 h bei 20 °C, gibt wenig Wasser zur Reaktionsmischung und filtriert über Kieselgel. Man erhält nach Abdestillieren des Lösungsmittels und Destillation des Rückstandes 1,6 g (22 % d.Th.) Thiophosphorsäure-O-ethyl-O-(2,2,3,3,4,4-hexafluorcyclopentyl)-S-propyl-triester mit einem Siedepunkt $Kp_{0,15}$ von 90-92 °C.

Beispiel 7

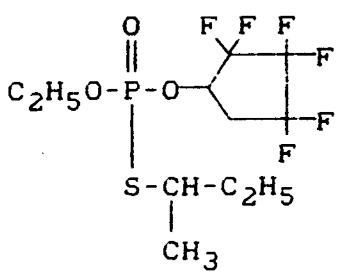

10,5 g (0,05 Mol) Thiophosphorsäure-S-(1-methyl-propyl)-ester werden in 200 ml Hexan gelöst und zunächst zwischen 0 und -5 °C mit 10 ml (0,07 Mol) Triethylamin, dann mit 2,3 g (0,05 Mol) Ethanol, gelöst in 25 ml Hexan, versetzt. Man rührt 2 Stunden bei 20 °C und tropft dann zunächst bei 20 °C 10 ml (0,07 Mol) Triethylamin, dann 10 g (0,052 Mol) 2,2,3,3,4,4-Hexafluorcyclopentanol-1 zur Reaktionsmischung. Zur Vervollständigung der Reaktion rührt man 24 h bei 20 °C, filtriert den Feststoff ab und destilliert das Lösungsmittel bei vermindertem Druck ab. Der Rückstand wird dann durch Filtration über Kieselgel (Cyclohexan:Essigsäureethylester = 7:3 Volumenteile) weiter gereinigt. Man erhält nach Abdestillieren des Lösungsmittels 18,0 g (98 % d.Th.) Thiophosphorsäure-O-(2,2,3,3,4,4-hexafluorcyclopentyl)-S-propyldiester-N-ethylamid mit einem Brechungsindex $n_D^{20}$ = 1,4265.

Analog werden hergestellt:

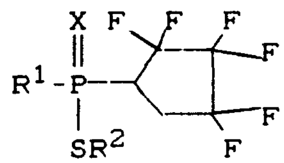

| Beispiel | $R^1$ | $R^2$ | X | phys. Daten |
|---|---|---|---|---|
| 9 | $C_2H_5NH$ | $CH(CH_3)C_2H_5$ | O | $n_D^{20}$ 1.4315 |
| 10 | $C_2H_5O$ | $n-C_3H_7$ | S | |
| 11 | $C_2H_5O$ | $CH(CH_3)C_2H_5$ | S | |
| 12 | $C_2H_5NH$ | $n-C_3H_7$ | S | |
| 13 | $C_2H_5NH$ | $CH(CH_3)C_2H_5$ | S | |

Beispiel 14 (Ausgangsstoffe der Formel III)

Stufe 1:

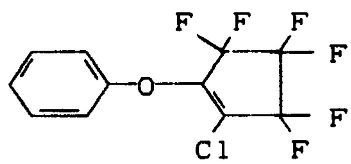

Die Herstellung erfolgt gemäß:
R. Stockel, Can. J. Chem., 53, 2302 (1975)

Stufe 2:

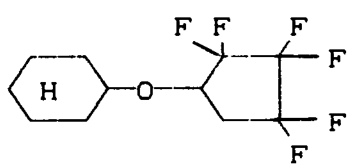

In einem 1,3 l Autoklaven werden 605 g (2 Mol) 2-Chlor-3,3,4,4,5,5-hexafluorcyclopentenyl-phenylether (aus Stufe 1) in 500 ml pH 9-Puffer (Bora/HCl) suspendiert und über 50 g Palladium-Kohle bei 70-80 °C mit 80 bar Wasserstoff hydriert. Der Katalysator wird abfiltriert und das Filtrat mit Dichlormethan extrahiert. Durch Destillation erhält man den 2,2,3,3,4,4-Hexafluorcyclopentyl-cyclohexylether.

| | |
|---|---|
| Kp. : | 86-89 °C |
| Ausbeute : | 502 g (91 % der Theorie) |
| $^1$H-NMR : | 1,08-1,54 ppm (m, 6H); 1,83 ppm (m, 4H); 2,34 ppm (m, 1H); 2,71 ppm (m, 1H); 3,48 ppm, (m, 1H); 4,20 ppm (m, 1H) |

$^{19}$F-NMR (gegen $CF_3COOH$): -33,5 ppm (m, 2F); -47,3 ppm (m, 2F); -55,0 ppm (m, 2F)

Aus dem Alkohol können die Alkalisalze nach den allgemeinen üblichen Salzbildungsmethoden erhalten werden.

Stufe 3:

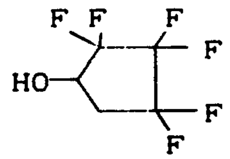

276 g (1 Mol) 2,2,3,3,4,4-Hexafluorcyclopentyl-cyclohexylether aus Stufe 2 werden in 300 ml konzentrierte Schwefelsäure bis zum Ende der Gasentwicklung eingetropft und das Spaltprodukt bei 130-140 °C abdestilliert. Das Gemisch aus dem gewünschten Alkohol und Cyclohexen wird redestilliert. Man erhält so durch Destillation das 2,2,3,3,4,4-Hexafluorcyclopentanol.

Ausbeute : 165 g (85 % der Theorie)
Kp. : 130-132 °C
$^1$H-NMR : 2,39 ppm (m, 1H); 2,72 ppm (m, 1H); 3,27 ppm (s, 1H), 4,38 ppm (m, 1H)
$^{19}$F-NMR : (gegen $CF_3COOH$): -32,2 ppm (m, 2F); -49,7 ppm, (m, 2F); -54,2 ppm (m, 2F)

Die biologische Wirkung der erfindungsgemäßen Verbindungen soll anhand der folgenden Beispiele erläutert werden.

Beispiel A

Phaedon-Larven-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z.B. die Verbindungen der Herstellungsbeispiele 1, 5, 6 und 7 bei einer beispielhaften Wirkstoffkonzentration von 0,01 % nach 3 Tagen eine Abtötung von 100 %.

Beispiel B

Grenzkonzentrations-Test / Bodeninsekten

Testinsekt: Diabrotica balteata - Larven im Boden
Lösungsmittel: 4 Gewichtsteile Aceton
Emulgator: 1 Gewichtsteil Alkylarylpolyglykol ether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,5 l Töpfe und läßt diese bei 20 °C stehen.

Sofort nach dem Ansatz werden je Topf 5 vorgekeimte Maiskörner gelegt. Nach 1 Tag werden in den

behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 % wenn noch genau so viele Testinsekten leben wie in der unbehandelten Kontrolle.

Bei diesem Test zeigte z.B. die Verbindung des Herstellungsbeispiels 6 bei einer beispielhaften Konzentration von 20 ppm einen Wirkungsgrad von 100 %.

Beispiel C

Grenzkonzentrations-Test / Bodeninsekten

Testinsekt: Phorbia antiqua-Larven im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und läßt diese bei Raumtemperatur stehen.

Nach 24 Stunden werden die Testinsekten in den behandelten Boden gegeben und nach weiteren 2 bis 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0%, wenn noch genau so viel Testinsekten leben wie bei der unbehandelten Kontrolle.

Bei diesem Test zeigten z.B. die Verbindungen der Herstellungsbeispiele 4 und 6 bei einer beispielhaften Konzentration von 20 ppm eine Abtötungszahl von 100 %.

Beispiel D

Grenzkonzentrations-Test / Nematoden

Testnematode: Meloidogyne incognita
Lösungsmittel: 4 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 25 °C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigte z.B. die Verbindung des Herstellungsbeispiels 6 bei einer beispielhaften Konzentration von 10 ppm einen Wirkungsgrad von 100 %.

Beispiel E

Grenzkonzentrations-Test

Testnematode: Globodera rostochiensis
Lösungsmittel: 4 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der

angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 20 ° C.

Nach sechs Wochen werden die Kartoffelwurzeln auf Zysten untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigte z.B. die Verbindung des Herstellungsbeispiels 6 bei einer beispielhaften Konzentration von 10 ppm einen Wirkungsgrad von 100 %.

## Beispiel F

Saatgutbehandlungs-Test / Bodeninsekten

| | |
|---|---|
| Testinsekt: | Diabrotica balteata - Larven im Boden |
| Testpflanze: | Zea mays |
| Lösungsmittel: | 1 Gewichtsteil Aceton |
| Trägermaterial: | Kaolin |

Zur Herstellung einer zweckmäßigen Wirkstoffzubereitung löst man den Wirkstoff in Aceton und vermischt 1 Gew.-Teil Wirkstoff/aceton mit 5 Gewichtsteilen Kaolin. Das Mais-Saatgut wird mit dieser Wirkstoffzubereitung mit den geforderten Aufwandmengen behandelt. Die Mais-Aussaat erfolgt in 0,5 l Töpfen mit standardisierten Boden bei 20 ° C Raumtemperatur.

Nach 1 Tag werden ca. 30 Diabrotica-Larven in jeden Topf gegeben. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie in der unbehandelten Kontrolle.

Bei diesem Test zeigte z.B. die Verbindung des Herstellungsbeispiels 6 bei einer beispielhaften Aufwandmenge von 2 g je kg Saatgut einen Wirkungsgrad von 100 %.

## Beispiel G

Saatgutbehandlungs-Test / Bodeninsekten

| | |
|---|---|
| Testinsekt: | Phorbia antiqua-Maden im Boden |
| Testpflanze: | Allium cepa |
| Lösungsmittel: | 1 Gewichtsteil Aceton |
| Trägermaterial: | Kaolin |

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Zwiebelsaatgut wird mit dieser Wirkstoffzubereitung mit den geforderten Aufwandmengen behandelt. Die Aussaat erfolgt in 0,5 l Töpfe mit standardisierten Böden bei 20 ° C Gewächshaustemperatur. Nach Auflauf der Zwiebeln werden diese mit Zwiebelfliegeneiern künstlich infiziert. Die Auswertung erfolgt nach 14 Tagen. Der Wirkungsgrad ist 100 %, wenn alle Zwiebelpflanzen stehenbleiben, 0 % wenn alle Testpflanzen (wie in der unbehandelten Kontrolle) vernichtet wurden.

Bei diesem Test zeigte z.B. die Verbindung des Herstellungsbeispiels 6 bei einer beispielhaften Aufwandmenge von 2 g je kg Saatgut einem 100 %igen Wirkungsgrad.

## Beispiel H

Fliegentest

| | |
|---|---|
| Testtiere: | Musca domestica, Stamm WHO(N) |
| Lösungsmitel: | 35 Gewichtsteile Ethylenglykolmonoethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben ($\phi$ 9,5 cm) pipettiert, die sich in Petrischalen entsprechender Größe befinden. Nach Trocknung der Filterscheiben werden 25 Testinsekten in die Petrischalen überführt und abgedeckt.

Nach 6 Stunden wird die Wirksamkeit der Wirkstoffzubereitung ermittelt. Die Wirksamkeit drückt man in % aus. Dabei bedeutet 100 %, daß alle Fliegen abgetötet wurden; 0 % bedeutet, daß keine Fliegen abgetötet wurden.

Bei diesem Test zeigten z.B. die Verbindungen der Herstellungsbeispiele 1,5 und 7 bei einer beispielhaften Wirkstoffkonzentration von 1000 ppm eine 100 %ige Wirksamkeit.

Beispiel J

Schabentest

Testinsekten: Periplaneta americana
Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben ($\phi$ 9,5 cm) pipettiert, die sich in Petrischalen entsprechender Größe befinden. Nach Trocknung der Filterscheiben werden 5 Testinsekten überführt und abgedeckt.

Nach 3 Tagen wird die Wirksamkeit der Wirkstoffzubereitung bestimmt. Die Wirksamkeit drückt man in % aus. Dabei bedeutet 100 %, daß alle Schaben abgetötet wurden; 0 % bedeutet, daß keine Schaben abgetötet wurden.

Bei diesem Test zeigten z.B. die Verbindungen der Herstellungsbeispiele 1 und 7 bei einer beispielhaften Wirkstoffkonzentration von 1000 ppm eine Wirksamkeit von 100 %.

Beispiel K

Blowfly-Larven-Test

Testtiere: Lucilia cuprina-Larven
Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird die Wirksamkeit der Wirkstoffzubereitung ermittelt. Dabei bedeutet 100 %, daß alle Blowfly-Larven abgetötet wurden.

Bei diesem Test zeigten z.B. die Verbindungen der Herstellungsbeispiele 1, 4, 5 und 7 bei einer beispielhaften Wirkstoffkonzentration von 1000 ppm eine Wirkung von 100 %.

Beispiel L

Kornkäfer-Test

Testinsekten: Sitophilus granarius
Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben ($\phi$ 9,5 cm) pipettiert, die sich in

Petrischalen entsprechender Größe befinden. Nach Trocknung der Filterscheiben werden 30 Testinsekten von S. granarius in die Petrischalen überführt und abgedeckt.

Nach 3 Tagen wird die Wirksamkeit der Wirkstoffzubereitung bestimmt. Die Wirksamkeit drückt man in % aus. Dabei bedeutet 100 %, daß alle Kornkäfer abgetötet wurden; 0 % bedeutet, daß keine Kornkäfer abgetötet wurden.

Bei diesem Test zeigten z.B. die Verbindungen der Herstellungsbeispiele 1 und 7 bei einer beispielhaften Wirkstoffkonzentration von 1000 ppm eine Wirkung von 100 %.

## Patentansprüche

**1.** O-Halogencyclopentyl-S-alkyl-(di) thiophosphor(phosphon)säureester-Derivate der allgemeinen Formel (I)

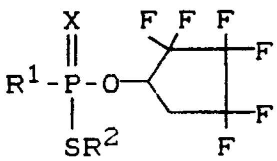

(I)

in welcher

$R^1$ für Alkyl, Alkoxy oder den Rest

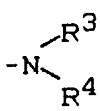

steht, wobei

$R^3$ für Wasserstoff oder Alkyl steht und

$R^4$ für Wasserstoff, Alkyl, -COH (Formyl) oder gegebenenfalls durch Halogen substituiertes -CO-Alkyl (Acyl) steht,

$R^2$ für Alkyl oder Alkoxyalkyl steht und

X für Sauerstoff oder Schwefel steht.

**2.** Verbindungen gemäß Anspruch 1, in welchen

$R^1$ für $C_1$-$C_6$-Alkyl, $C_1$-$C_6$-Alkoxy oder $-NR^3R^4$ steht, worin

$R^3$ für Wasserstoff oder $C_1$-$C_6$-Alkyl steht und

$R^4$ für Wasserstoff, $C_1$-$C_6$-Alkyl, HCO- (Formyl) oder gegebenenfalls durch Halogen substituiertes -CO-$C_1$-$C_4$-Alkyl (Acyl) steht,

$R^2$ für $C_1$-$C_6$-Alkyl oder $C_1$-$C_6$-Alkoxy-$C_1$-$C_6$-alkyl steht und

X für Sauerstoff oder Schwefel steht.

**3.** Verbindungen gemäß Anspruch 1, in welchen

$R^1$ für $C_1$-$C_4$-Alkyl, $C_1$-$C_4$-Alkoxy oder $-NR^3R^4$, worin

$R^3$ für Wasserstoff oder $C_1$-$C_4$-Alkyl steht und

$R^4$ für Wasserstoff, $C_1$-$C_4$-Alkyl, HCO- oder gegebenenfalls durch Halogen substituiertes -CO-$C_1$-$C_4$-Alkyl steht,

$R^2$ für $C_1$-$C_4$-Alkyl oder $C_1$-$C_4$-Alkoxy-$C_1$-$C_4$-alkyl steht und

X für Sauerstoff oder Schwefel steht.

**4.** Verbindungen gemäß Anspruch 1, in welchen

$R^1$ für $C_1$-$C_3$-Alkyl, Methoxy, Ethoxy, $-NHCH_3$ oder $-NHC_2H_5$ steht,

$R^2$ für $C_3$-$C_4$-Alkyl steht und

X für Sauerstoff oder Schwefel steht.

**5.** Verbindungen gemäß den Ansprüchen 1 bis 4, wobei X für Sauerstoff steht.

6. Verfahren zur Herstellung von O-Halogencyclopentyl-S-alkyl-(di)thiophosphor(phosphon)säureester-Derivaten der allgemeinen Formel (I)

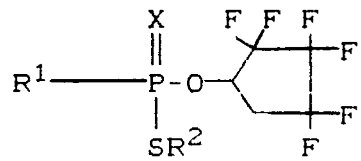

(I)

in welcher

$R^1$ für Alkyl, Alkoxy oder den Rest

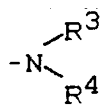

steht, wobei

$R^3$ für Wasserstoff oder Alkyl steht und

$R^4$ für Wasserstoff, Alkyl, -COH (Formyl) oder gegebenenfalls durch Halogen substituiertes -CO-Alkyl (Acyl) steht,

$R^2$ für Alkyl oder Alkoxyalkyl steht und

X für Sauerstoff oder Schwefel steht,

dadurch gekennzeichnet, daß man

a) Thio- oder Dithiophosphorsäureesterhalogenide der Formel (II)

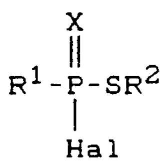

(II)

in welcher

$R^1$, $R^2$ und X die oben angegebene Bedeutung haben und

Hal für Halogen steht,

mit Cyclopentanol-Derivaten der Formel (III)

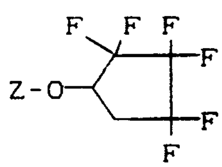

(III)

in welcher

Z für Wasserstoff oder ein Äquivalent eines Alkalimetallions steht, gegebenenfalls in Gegenwart von Verdünnungsmitteln und gegebenenfalls in Gegenwart von Basen umsetzt,

oder

b) zur Herstellung von Verbindungen der Formel (I), in welchen $R^1$ für Alkoxy oder $-NR^3R^4$ steht, in einem ersten Reaktionsschritt Säurehalogenide der Formel (IV)

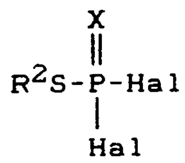

(IV)

in welcher

X, $R^2$ und Hal die oben angegebene Bedeutung besitzen,

mit Verbindungen der Formel

$R^5$-H (V)

in welcher

$R^5$ für Alkoxy oder den Rest $-NR^3R^4$ steht, wobei $R^3$ und $R^4$ die oben angegebene Bedeutung haben,

gegebenenfalls in Gegenwart von Lösungsmitteln und gegebenenfalls in Gegenwart von Basen umsetzt und das erhaltene Produkt der Formel (VI)

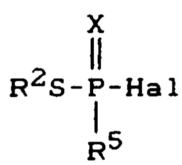

(VI)

in welcher

X, $R^2$, $R^5$ und Hal die oben angegebene Bedeutung haben,

anschließend in einem zweiten Reaktionsschritt mit Verbindungen der Formel (III) gegebenenfalls in Gegenwart von Verdünnungsmitteln und gegebenenfalls in Gegenwart von Basen, umsetzt,

oder

c) in einem ersten Reaktionsschritt Verbindungen der Formel (VII)

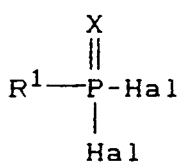

(VII)

in welcher

$R^1$, X und Hal die oben angegebene Bedeutung haben,

mit Verbindungen der Formel (III), gegebenenfalls in Gegenwart von Verdünnungsmitteln und gegebenen falls in Gegenwart von Basen, umsetzt und die erhaltenen Verbindungen der Formel (VIII)

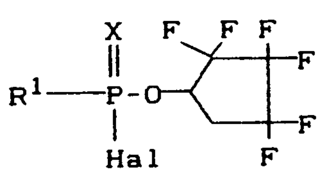

(VIII)

in welcher

$R^1$, X und Hal die oben angegebene Bedeutung haben,

in einem zweiten Reaktionsschritt mit Verbindungen der Formeln (IXa) oder (IXb)

$Z^1$-SH (IXa)

oder

$(Z^1)_2S$ (IXb)

in welcher
$Z^1$ für ein Äquivalent eines Alkalimetallions steht,
gegebenenfalls in Gegenwart eines Verdünnungsmittels, umsetzt und die erhaltenen Verbindungen der Formel (X)

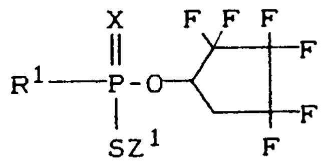

(X)

in welcher
$R^1$, X und $Z^1$ die oben angegebene Bedeutung haben,
mit Verbindungen der Formel (XI)

$R^2$-Hal (XI)

in welcher
$R^2$ und Hal die oben angegebene Bedeutung haben,
gegebenenfalls in Gegenwart eines Verdünnungsmittels und gegebenenfalls in Gegenwart einer Base, umsetzt,

oder

d) zur Herstellung von Verbindungen der Formel (I), in welchen $R^1$ für Alkyl und X für Schwefel stehen, in einem ersten Reaktionsschritt, Verbindungen der Formel (XII)

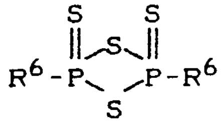

(XII)

in welcher
$R^6$ für Alkyl steht,
mit Cyclopentanol-Derivaten der Formel (III), gegebenenfalls in Gegenwart von Verdünnungsmitteln und gegebenenfalls in Gegenwart von Basen, umsetzt, und die erhaltenen Verbindungen der Formel (XIII)

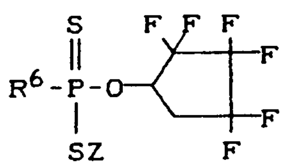

(XIII)

in welcher

$R^6$ und Z die oben angegebene Bedeutung haben,
in einem zweiten Schritt mit Verbindungen der Formel (XI), gegebenenfalls in Gegenwart von Verdünnungsmitteln und gegebenenfalls in Gegenwart von Basen, umsetzt,
oder
e) zur Herstellung von Verbindungen der Formel (I), in welchen $R^1$ für $-NR^3R^4$ steht, in einem ersten Schritt Verbindungen der Formel (IV) mit Verbindungen der Formel (III), gegebenenfalls in Gegenwart von Verdünnungsmitteln und gegebenenfalls in Gegenwart von Basen, umsetzt und in einem zweiten Schritt die erhaltenen Verbindungen der Formel (XIV)

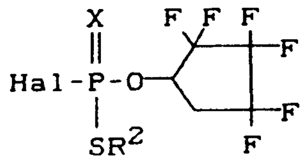

(XIV)

in welcher
X, $R^2$ und Hal die oben angegebene Bedeutung haben,
mit Verbindungen der Formel (XV)

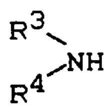

(XV)

in welcher
$R^3$ und $R^4$ die oben angegebene Bedeutung haben,
gegebenenfalls in Gegenwart eines Verdünnungsmittels und gegebenenfalls in Gegenwart einer Base, umsetzt,
oder
f) zur Herstellung von Verbindungen der Formel (I), in welcher $R^4$ für Formyl oder Acyl steht, Verbindungen der Formel (XVI)

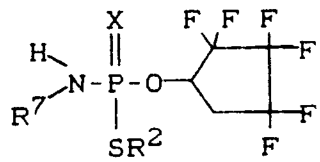

(XVI)

in welcher
X und $R^2$ die oben angegebene Bedeutung haben und
$R^7$ für Wasserstoff oder Alkyl steht,
mit Verbindungen der Formel (XVII)

$R^8$-CO-Y (XVII)

in welcher
$R^8$ für Wasserstoff oder gegebenenfalls durch Halogen substituiertes Alkyl steht und
Y für Halogen oder eine andere bei Acylierungsreaktionen abgehende Gruppe steht,
gegebenenfalls in Gegenwart eines Verdünnungsmittels oder gegebenenfalls in Gegenwart einer Base, umsetzt.

**7.** Schädlingsbekämpfungsmittel, gekennzeichnet durch einen Gehalt an mindestens einer Verbindung der Formel (I), gemäß den Ansprüchen 1 oder 6.

**8.** Verwendung von Verbindungen der Formel (I) gemäß den Ansprüchen 1 oder 6 zur Bekämpfung von Schädlingen.

**9.** Verfahren zur Bekämpfung von Schädlingen, dadurch gekennzeichnet, daß man Verbindungen der Formel (I) gemäß den Ansprüchen 1 oder 6 auf Schädlinge und/oder ihren Lebensraum einwirken läßt.

**10.** Verfahren zur Herstellung von Schädlingsbekämpfungsmitteln, dadurch gekennzeichnet, daß man Verbindungen der Formel (I) gemäß den Ansprüchen 1 oder 6 mit Streckmitteln und/oder oberflächenaktiven Mitteln vermischt.

**11.** Verbindungen der Formel (XVIII)

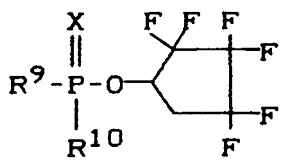

(XVIII)

in welcher

(a)

$R^9$ die Bedeutung von $R^1$ hat und

$R^{10}$ für Halogen oder $SZ^1$ steht oder

(b)

$R^9$ für Halogen steht und $R^{10}$ für $SR^2$ steht,

wobei $R^1$ und $R^2$ die in Anspruch 1 angegebene Bedeutung haben und

$Z^1$ für ein Äquivalent eines Alkalimetallions steht.

**12.** Verbindungen der Formel (III)

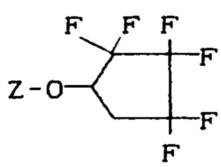

(III)

in welcher

Z für Wasserstoff oder ein Äquivalent eines Alkalimetallions steht.